# DEMANDE DE BREVET EUROPEEN

(11) **EP 1 440 681 A1**
(43) Date de publication de la demande: **28.07.2004**
(21) Numéro de dépôt: 04290062.1
(22) Date de dépôt: 09.01.2004
(51) Int. Cl.: A61K 7/13, A61K 7/00, A61K 7/06

(54) **Utilisation de nanoparticules semiconductrices luminescentes en cosmétique**

(30) Priorité: 27.01.2003 FR 0300860
(71) Demandeur: L'OREAL, 75008 Paris (FR)
(72) Inventeur: Gourlaouen, Luc, 92600 Asnieres (FR); Lee, Kenneth, 75017 Paris (FR)
(74) Mandataire: Wattremez, Catherine

(57) **Abrégé**

La présente invention qui a pour objet l'utilisation de nanoparticules semiconductrices luminescentes capables d'émettre sous l'action d'une excitation lumineuse un rayonnement présentant une longueur d'onde comprise entre 400 nm et 700 nm, dans le domaine de la cosmétique.

En particulier, la présente invention a pour objet l'utilisation de nanoparticules pour la coloration des matières kératiniques tels que les cheveux.

## Description

L'invention a pour objet l'utilisation dans le domaine de la cosmétique de nanoparticules semi-conductrices luminescentes, en particulier des nanoparticules contenant du sulfure de cadnium ou du sélénure de cadnium. L'invention a aussi pour objet l'utilisation de telles nanoparticules pour la coloration des matières kératiniques.

Dans le domaine de la teinture des fibres kératiniques, en particulier des fibres kératiniques humaines telles que les cheveux, il existe deux modes de coloration qui présentent chacun leurs avantages et leurs inconvénients :
- La *coloration directe* ou *coloration semi-permanente* consiste à amener la couleur par une molécule colorée qui s'adsorbe à la surface des fibres kératiniques et/ou pénètre par diffusion dans les couches superficielles de celles-ci. Les temps de pose sont généralement assez courts et les conditions douces de teinture préservent l'intégrité des fibres kératiniques, mais les colorations obtenues par ce mode de teinture résistent mal au lavage et s'estompent après seulement 4 ou 5 shampooings. De plus, les gammes des nuances obtenues sont en général réduites.
- La *coloration d'oxydation* ou *coloration permanente* met en oeuvre la condensation oxydative de molécules incolores ou faiblement colorées, appelées bases d'oxydation, telles que des ortho ou para-phénylènediamines, des ortho ou para-aminophénols, des composés hétérocycliques en présence d'un agent oxydant. Cette réaction conduit à la formation de composés colorés polymères dans les fibres kératiniques. Le principal avantage de la teinture d'oxydation réside dans la longévité des colorations obtenues, en particulier dans la résistance aux lavages et aux agents extérieurs tels que la lumière, les intempéries, les ondulations permanentes, la transpiration et les frottements, ainsi que dans l'obtention d'une large palette de nuances. Cependant, les conditions chimiques de teinture, telles que le pH et un milieu oxydant, entraînent une dégradation des fibres kératiniques. Par ailleurs, ce mode de teinture nécessite des temps de pose relativement longs.

Il est connu d'utiliser des nanoparticules inorganiques luminescentes semi-conductrices, en particulier en sélénure de cadnium ou en sulfure de cadnium pour le marquage biologique de cellules, en particulier dans le brevet US 5 990 479.

Le but de la présente invention est de fournir de nouvelles compositions tinctoriales pour la teinture de fibres kératiniques ne présentant pas les inconvénients de celles de l'art antérieur. En particulier, le but de la présente invention est de fournir un nouveau système de coloration qui présente à la fois les avantages de la ténacité, en particulier aux lavages répétés, du respect de la fibre capillaire et qui permettent d'obtenir une gamme variée de nuances.

Ce but est atteint avec la présente invention qui a pour objet l'utilisation de nanoparticules semiconductrices luminescentes capables d'émettre sous l'action d'une excitation lumineuse, un rayonnement présentant une longueur d'onde comprise entre 400 nm et 700 nm dans le domaine de la cosmétique.

Les nanoparticules utiles pour la présente invention permettent d'obtenir en particulier dans le domaine de la coloration des matières kératiniques une coloration ou un éclaircissement optique tenace. Dans le cas particulier des fibres kératiniques, en particulier des cheveux, on obtient une coloration ou un éclaircissement tenace et sans dégradation des fibres kératiniques.

Au sens de la présente invention, on entend par coloration ou éclaircissement optique un effet visuel de coloration ou d'éclaircissement des fibres kératiniques colorées, naturellement ou artificiellement, sans utiliser de composés détruisant les pigments colorés, naturels ou artificiels, présents dans les fibres kératiniques.

Ces nanoparticules présentent la particularité de présenter des spectres d'émission de la couleur beaucoup plus étroits que la plupart des colorants ou pigments organiques utilisés en coloration capillaire. On obtient ainsi des colorations plus pures.

De plus, la couleur émise par ces nanoparticules varie en fonction du diamètre de celles ci. Ainsi, on peut obtenir des gammes de couleurs très variées en utilisant dans les compositions une ou plusieurs tailles de nanoparticules. Ces nanoparticules possèdent aussi la particularité d'émettre des couleurs très intenses.

Selon un mode de réalisation particulier de l'invention, les nanoparticules comprennent au moins un métal choisi parmi Zn, Cd, Hg et au moins un métal choisi parmi S, Se et Te. De préférence, ces nanoparticules comprennent du séléniure de cadmium ou du sulfure de cadmium.

Dans les nanoparticles utiles dans la présente invention, les métaux présents peuvent être répartis de façon homogène. Les nanoparticules peuvent aussi comprendre un coeur comprenant un ou plusieurs métaux et une ou plusieurs couches recouvrant ce coeur, comprenant un ou plusieurs métaux différents de ceux présents dans le coeur. Ces nanoparticules sont de préférence constituées d'un coeur constitué d'un ou plusieurs métaux et d'une ou plusieurs couches recouvrant ce coeur, constituées d'un ou plusieurs métaux différents de ceux constituant le coeur. Ces nanoparticules sont connues dans la littérature sous la forme de nanoparticules à coeur et à coque (core/shell).

Selon ce mode de réalisation particulier, les nanoparticules peuvent présenter un coeur en sélénure de cadnium recouvert d'une couche de sulfure de zinc.

Les nanoparticules peuvent aussi être recouvertes d'une ou plusieurs couches additionnelles organiques et/ou inorganiques, présentant de préférence une affinité pour les cheveux. A titre d'exemple de couche organique, on peut citer les couches obtenues à partir de polyéthylène glycol, polyuréthane, de dextran, de polyacrylique, de polyvinylpyrolidone, de polyvinylcaprolactone ou un mélange de ces matériaux.

A titre de couche inorganique, on peut citer à titre d'exemple, les couches obtenues à partir d'alumine, de silice ou d'argile ou un mélange de ces matériaux.

Ces couches peuvent être obtenues par procédé sol-gel à partir d'organosilane. Ces couches qui sont obtenues par encapsulation des nanoparticules peuvent être réalisées par divers procédés tels que la précipitation contrôlée, la séparation de phase, la polymérisation en émulsion, la polycondensation interfaciale, la polycondensation in situ.

Pour plus de détails, de tels procédés d'encapsulation sont décrits dans « Microencapsulation Methods and industrial Applications » (ISBN 0-8247-9703-5).

La capsule peut être formée par tous composés inorganiques, plus spécifiquement par un oxyde métallique ou un polymère organométallique et encore plus spécifiquement par un oxyde métallique ou un polymère organométallique obtenus par procédé sol gel tels que les oxydes métalliques ou les polymères organométalliques synthétisés par polycondensation d'un seul ou d'un mélange d'alcoxy simple ou mixte de silicium, d'aluminium, de bore, de lithium, de magnésium, de sodium, de titane et/ou de zirconium. Pour plus de détails sur la nature des précurseurs et les mécanismes réactionnels, on peut se référer à l'ouvrage « Sol Gel Science» publié par C.J Brinker et G.W. Scherer aux éditions Academic Press (ISBN 0-12-134970-5).

Ces couches additionnelles peuvent être greffées de façon covalente ou être adsorbées à la surface de la nanoparticules.

Selon un mode de réalisation différent, les nanoparticules peuvent être incorporées dans des microbilles de polymère, le polymère pouvant être hydrophile, hydrophobe, amphiphile, ionique ou non-ionique. A titre de polymère, on peut citer les polystyrènes selon les procédés décrits dans : « Quantum-dot-tagged microbeads for multiplexed optical coding of biomolecules », Mingyong Han, Nature Biotechnology Vol. 19, PP. 631-635 July 2001.

Les nanoparticules utiles dans la présente invention peuvent présenter des tailles variant de 1 à 100 nm, de préférence entre 1 et 50 nm. De façon particulièrement préférée, ces nanoparticules présentent un diamètre compris entre 1 et 20 nm.

Les nanoparticules sont connus de la littérature. En particulier, ces nanoparticules peuvent être fabriqués selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size séries of highly luminescent nanocristallites" *Journal of phisical chemistry B, vol 101, 1997, pp 9463-9475.* et Peng, Xiaogang et al, "Epitaxial Growth of highly Luminescent CdSe/CdS core/shell nanocrystals with photostability and electronic accessibility" *Journal of the American Chemical Society, vol 119, N°30, pp 7019-7029.* Les nanoparticules utiles dans la présente invention sont aussi connues sous le nom de "Quantum dots".

A titre d'exemple, on peut citer les nanoparticules suivantes :

| Type de nanoparticules | Taille | Couleurs | Concentration de la solution |
|---|---|---|---|
| CdSe | 2,2nm | Vert | 0,5mg/ml |
| CdSe | 3,4nm | Jaune | 0,5mg/ml |
| CdSe | 4,0nm | Orange | 0,5mg/ml |
| CdSe | 4,7nm | Rouge orangé | 0,5mg/ml |
| CdSe | 5,6nm | Rouge | 0,5mg/ml |
| CdSe/ZnS | 4,3nm | Vert | 0,5mg/ml |
| CdSe/ZnS | 4,8nm | Jaune vert | 0,5mg/ml |
| CdSe/ZnS | 5,4nm | Jaune | 0,5mg/ml |
| CdSe/ZnS | 6,3nm | Orange | 0,5mg/ml |
| CdSe/ZnS | 7,2nm | Rouge | 0,5mg/ml |

Ces nanoparticules sont fournies par la société EVIDENT Technologies Les nanoparticules CdSe sont des nanoparticules uniformes qui contiennent uniquement de CdSe. Les nanoparticules CdSe/ZnS ont des structures core/shell avec un « core » de CdSe et une « shell » de ZnS.

Selon un mode de réalisation particulier, les nanoparticules peuvent être utilisées pour la coloration des matières kératiniques, en particulier des fibres kératiniques telles que les cheveux.

La présente invention a ainsi pour objet une composition pour la coloration des matières kératiniques qui comprend au moins les nanoparticules décrites précédemment, dans un milieu cosmétique approprié.

La composition de la présente invention est en particulier destinée à la coloration des cheveux. Dans ce cas, la composition peut se présenter sous forme de teinture, de shampoing, d'après shampoing, de laque ou de composition de mise en forme des cheveux. L'homme du métier définira sans difficulté à partir de ces connaissances générales la composition du milieu en fonction de la nature de la composition de coloration.

Selon un mode de réalisation particulièrement préféré, la composition selon l'invention est une composition tinctoriale destinée à la teinture des fibres kératiniques qui comprend dans un milieu de teinture approprié des nanoparticules telles que définies précédemment.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

La composition selon la présente invention comprend de préférence, en plus des nanoparticules, un agent tensio-actif.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 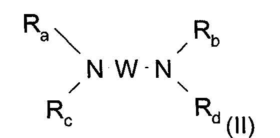 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un procédé de traitement des fibres kératiniques et en particulier des fibres kératiniques humaines telles que les cheveux, qui comprend l'application sur les fibres d'une composition selon la présente invention.

Selon un mode de réalisation particulier, le procédé de l'invention est un procédé de teinture des fibres kératiniques. La composition de l'invention peut être appliquée sur fibres sèches ou humides. L'application peut être suivie d'une étape de lavage et/ou de rinçage des fibres kératiniques.

Les exemples qui suivent servent à illustrer l'invention sans toutefois présenter un caractère limitatif.

### EXEMPLE

A une mèche de cheveux de 30 mg de cheveux naturels 90% blancs, on ajoute 1 ml de solution de nanoparticules de SeCd de 4,0 nm de diamètre moyen, on laisse poser pendant 5 minutes.

On égoutte la mèche de cheveux, on la sèche et on observe la couleur à la lumière du jour. La mèche est colorée en orange.

## Revendications

1. Utilisation de nanoparticules semi conductrices luminescentes capables d'émettre sous excitation lumineuse, un rayonnement présentant une longueur d'onde comprise entre 400 nm et 700 nm, dans le domaine de la cosmétique.

2. Utilisation selon la revendication 1 dans laquelle les nanoparticules comprennent au moins un métal choisi parmi Zn, Cd, Hg et au moins un métal choisi parmi S, Se et Te.

3. Utilisation selon la revendication 1 ou 2 dans laquelle les nanoparticules comprennent du séléniure de cadmium ou du sulfure de cadmium.

4. Utilisation selon la revendication 1 ou 2 dans laquelle les nanoparticules comprennent un coeur comprenant un ou plusieurs métaux et une ou plusieurs couches recouvrant ce coeur comprenant un ou plusieurs métaux différents de ceux présents dans le coeur.

5. Utilisation selon la revendication 4 dans laquelle les nanoparticules présentent un coeur en sélénure de cadnium recouvert d'une couche de sulfure de zinc.

6. Utilisation selon l'une quelconque des revendications 1 à 5 dans laquelle les nanoparticules sont recouvertes d'une ou plusieurs couches additionnelles organiques et/ou inorganiques.

7. Utilisation selon la revendication 6 dans laquelle la ou les couches organiques comprennent du polyéthylène glycol, du polyuréthane, du dextran, du polyacrylique, du polyvinylpyrolidone, du polyvinylcaprolactone ou un mélange de ces matériaux, de préférence du dextran ou du polyéthylène glycol ou un mélange de ces matériaux.

8. Utilisation selon la revendication 6 dans laquelle la ou les couches inorganiques comprennent de l'alumine, de la silice ou de l'argile ou un mélange de ces matériaux, de préférence de la silice ou de l'alumine ou un mélange de ces matériaux.

9. Utilisation selon l'une quelconque des revendications 1 à 8 dans laquelle les nanoparticules sont incorporées dans des microbilles de polymère.

10. Utilisation selon l'une quelconque des revendications 1 à 9 dans laquelle la taille des nanoparticules est comprises entre 1 et 100 nm, de préférence entre 1 et 50 nm.

11. Utilisation selon l'une quelconque des revendications 1 à 10 pour la coloration des matières kératiniques.

12. Utilisation selon la revendication 11 pour la coloration des cheveux.

13. Composition de coloration comprenant dans un milieu de teinture approprié des nanoparticules telles que définies selon l'une quelconque des revendications 1 à 10.

14. Composition selon la revendication 13 comprenant de plus au moins un agent tensio-actif.

15. Composition selon la revendication 13 sous forme de teinture, de shampooing, d'après-shampoing, de laque ou de composition de mise en forme du cheveu.

16. Procédé de coloration des fibres kératiniques qui comprend l'application sur ces fibres d'une composition telle que définie aux revendications 13 à 15 comprenant les nanoparticules.

17. Procédé selon la revendication 16 pour la teinture des fibres kératiniques.
